# EUROPEAN PATENT APPLICATION

(11) **EP 3 447 066 A1**
(43) Date of publication of application: **27.02.2019**
(21) Application number: 17186995.1
(22) Date of filing: 21.08.2017
(51) Int. Cl.: C07K 14/52, A61K 38/00, A61K 39/00

(54) **NEW CCL2 PEPTIDES FOR USE IN CANCER THERAPY**

(71) Applicant: Herlev Hospital, 2730 Herlev (DK)
(72) Inventor: ANDERSEN, Mads Hald, 2850 NÆRUM (DK)
(74) Representative: Kjerrumgaard, Lars Bo

(57) **Abstract**

The present invention relates to a peptide compound of CCL2/MCP-1 selected from a peptide fragment, a functional homologue, and a functional analogue, as well as to a nucleic acid, such as DNA or RNA, encoding the peptide compound, a vector, such as a virus vector, and a host cell, such as mammalian cell, comprising the vector. The peptide compound, nucleic acid, vector and host cell of the present invention are in particular, useful for the treatment or prevention of a cancer characterized by expression of CCL2.

## Description

### Technical field

The present invention relates to novel peptide compounds, such as fragments of CCL2 (MCP-1), as well as compositions, uses, and kit-of-parts comprising these peptide compounds. Furthermore, the invention concerns nucleic acids, vectors, and host cells expressing said peptide compounds, for use in a method for treatment or prevention of a cancer, either alone or when administered simultaneously or sequentially with an additional cancer therapy.

### Background Art

Infiltration of leukocytes is one of the hallmarks of the inflammatory response. Among the leukocyte populations, neutrophils are the first to infiltrate, followed by monocytes and lymphocytes, suggesting the presence of mediators that specifically recruit these cell types. Cancer cells and host stromal cells in the tumor microenvironment including endothelial cells, fibroblasts, mesenchymal stem cells and infiltrating leukocytes produce a wide range of chemokines that exert numerous biological functions during tumor progression and metastasis (1, 2). Chemokines are a family of small and structurally related chemotactic cytokines that interact with their chemokine receptors to orchestrate cell migration and homing in the body. Of these, Chemokine C-C Motif Ligand 2 (CCL2) (also known as Monocyte Chemotactic Protein-1 (MCP-1)), together with the only known receptor CCR2, have been shown to play key roles in cancer. The CCL2/CCR2 axis has been described to influences metastasis by sustaining cancer cell proliferation and survival, angiogenesis, cancer cell migration and invasion and inducing deleterious inflammation (3-5).

Since a number of tumor cell lines constitutively produce CCL2 in vitro, tumor cells were originally thought to be the primary source of CCL2 in established tumors. Breast, ovarian, and gastric human tumors cultured in vitro secrete CCL2, and myeloid-derived suppressor cells (MDSCs) from these patients express the cognate CCR2 receptor and migrate towards these chemokines (6). However, recent studies strongly suggested that stromal cells are additional important sources of CCL2 in some tumors (7, 8). There are three potential mechanisms by which CCL2 production is increased in tumors; (1) tumor cells constitutively produce CCL2, (2) tumor cells produce CCL2 in response to stimuli, and (3) stromal cells produce CCL2 in response to stimuli, such as a tumor cell product(s).

CCR2 is expressed on monocytes and macrophages. Monocytes and macrophages migrate toward inflamed tissues under the influence of CCL2) (5). Hence, when CCL2 and CCR2 are upregulated, they promote macrophage accumulation and inflammation (9). Clinicopatho-logically, the correlation between macrophage accumulation and the CCL2 level has been demonstrated in a broad spectrum of human tumors including breast, prostate, ovarian, and non-small lung cancers (4, 10-12). CCL2 is in addition one of the main chemokines implicated in MDSCs migration to tumors (7, 8, 13). Hence, there is evidence suggesting that MDSCs are recruited to the tumor site in response to CCL2 (8). CCL2 expression increases with progression of colorectal cancer in humans and deletion of CCL2 in a spontaneous mouse model of colorectal cancer reduced the numbers of colonic MDSC (14). In addition, it has been described that CCL2 is overexpressed in human HCCs and is associated with a poor prognosis for patients.

Tumor-derived CCL2 acts as a potent factor for Th2 polarization and shifts monocytes toward the M2-polarized macrophages (15). CCL2 modulates macrophage polarization with macrophage colony stimulating factor-1 (M-CSF or CSF-1) (16). Tumor-associated macrophages represent the most abundant infiltrating immune population in human ovarian tumors and ascites (17). Ovarian tumors recruit circulating monocytes and induce differentiation into TAMs via expression of factors such as CCL2. Thus, CCL2 is overexpressed in ovarian tumor cells and cell lines, but surprisingly not in TAMs (11). Interestingly, expression of its receptor, CCR2 is defective in TAMs derived from ovarian cancer patients (18). This may reflect a mechanism by which ovarian tumors retain recruited macrophages in the tumor microenvironment. Expression of CSF-1 is higher in malignant ovarian tumors compared to borderline and benign tumors (19).

### Summary of the invention

The present inventor has identified new immunogenic epitopes from the signal peptide of MCP-1 (human MCP-1) identified by SEQ ID NO 1 herein. In the present study, the possibility of using stimulation of CCL2-specific T cells to target the recruitment and composition of regulatory cells to the microenvironment has been investigated.

In one aspect, the present invention concerns a peptide compound of MCP-1 selected from:
a) a peptide fragment of MKVSAALLCLLLIAATFIPQGLA (MCP-1(1-23)) (SEQ ID NO 1) consisting of a consecutive sequence of from 6 to 22 amino acids,
b) a functional homologue having at least 70%, 80%, 90%, or 95% identity to SEQ ID NO 1 or the peptide fragment of a), and
c) a functional analogue wherein at least one amino acid has been deleted, inserted and/or substituted in SEQ ID NO 1 or the peptide fragment of a),
and wherein the C-terminal amino acid of any one of a), b) or c) also comprises the amide; or a pharmaceutically acceptable salt thereof.

In an embodiment, the peptide compound is selected from a) a peptide fragment of SEQ ID NO 1 consisting of a consecutive sequence of from 6 to 22 amino acids, wherein the C-terminal amino acid also comprises the amide; or a pharmaceutically acceptable salt thereof.

In another embodiment, the peptide fragment consists of a consecutive sequence in the range of from 8 to 20 amino acids, 8 to 16 amino acids, 8 to 12 amino acids, or 9-12 amino acids.

In a further embodiment, the peptide fragment is selected from the group consisting of MCP-1(1-18), MCP-1(5-18), MCP-1(6-18), MCP-1(7-18), MCP-1(9-18), MCP-1(5-23), MCP-1(6-23), MCP-1(7-23), MCP-1(9-23), and MCP-1(10-23).

Thus, in a further aspect the present invention concerns a peptide compound of MCP-1 selected from:
a) MCP-1(5-18) or a peptide fragment of AALLCLLLIAATFI (MCP-1(5-18)) (SEQ ID NO 7) consisting of a consecutive sequence of from 6 to 13 amino acids,
b) a functional homologue having at least 70%, 80%, 90%, or 95% identity to SEQ ID NO 7 or the peptide fragment of a), and
c) a functional analogue wherein at least one amino acid has been deleted, inserted and/or substituted in SEQ ID NO 7 or the peptide fragment of a),
and wherein the C-terminal amino acid of any one of a), b) or c) also comprises the amide; or a pharmaceutically acceptable salt thereof.

In a further embodiment, the consecutive sequence comprises a sequence selected from the group consisting of CLLLIAATF, ALLCLLLIA, LLCLLLIAA, LLLIAATFI, and AALLCLLLI.

In a still further embodiment, the peptide fragment under a), the functional homologue under b), or the functional analogue under c) is capable of activating T-cells, such as CD4 and CD8 T-cells. Typically, the activation is determined by the ELISPOT assay described herein.

In a further embodiment, the peptide compound is selected from the group consisting of CLLLIAATF, ALLCLLLIA, LLCLLLIAA, LLLIAATFI, and AALLCLLLI.

In a further aspect, the present invention relates to a nucleic acid, such as DNA or RNA, encoding the peptide compound of any one of the preceding embodiments or aspect.

In a still further aspect, the present invention relates to a vector, such virus vector, comprising the nucleic acid of the present invention.

In a further aspect, the present invention relates to a host cell, such as mammalian cell, comprising the vector of claim the present invention.

In a still further aspect, the present invention relates to a composition comprising the MCP-1(1-23) peptide (SEQ ID NO 1) or the peptide compound of the present invention or the nucleic acid of the present invention or the vector of the present invention or the host cell of the present invention, optionally together with a pharmaceutically acceptable additive, such as a carrier or adjuvans.

In a further aspect, the present invention relates to an immunotherapeutic composition comprising
a) the MCP-1 (1-23) peptide (SEQ ID NO 1) or the peptide compound of the present invention or the nucleic acid of the present invention or the vector of the present invention or the host cell of the present invention; and
b) an adjuvant;
for use as a medicament.

In an embodiment, the immunotherapeutic composition is for use in a method for treatment or prevention of a disease, disorder or condition selected from cancer, such as a tumor forming cancer disease.

In a further embodiment, the adjuvant is selected from the group consisting of bacterial DNA based adjuvants, oil/surfactant based adjuvants, viral dsRNA based adjuvants, imidazochinilines, a Montanide ISA adjuvant.

In a further aspect, the present invention relates to a kit-of-parts comprising:
a) the immunotherapeutic composition of the present invention, and
b) a composition comprising at least one second active ingredient, selected from an immunostimulating compound, such as an interleukin, e.g. IL-2 and or IL-21, an anti-cancer agent, such as a chemotherapeutic agent, e.g. Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotec-an, Lenalidomide, Leucovorin, Mechlorethamine, Melphalan, Mercaptopurine, Methotrexate, Mitoxantrone, nivolumab, Oxaliplatin, Paclitaxel, pembrolizumab, Pemetrexed, Revlimid, Temozolomide, Teniposide, Thioguanine, Valrubicin, Vinblastine, Vincristine, Vindesine and Vinorelbine.

In an embodiment, the provided compositions are to be administered simultaneously or sequentially.

In a further aspect, the present invention relates to a method of treating a clinical condition characterized by expression of human MCP-1 or the MCP-1(1-23) peptide (SEQ ID NO 1), the method comprising administering to an individual suffering from said clinical condition an effective amount of the peptide compound of the present invention or the nucleic acid of the present invention or the vector of the present invention or the host cell of the present invention.

In a still further aspect, the present invention relates to a method of treating or preventing cancer in a patient, the method comprising administering to the cancer patient an effective amount of the peptide compound of the present invention or the nucleic acid of the present invention or vector of the present invention or host cell of the present invention.

In a further aspect, the present invention relates to use of the peptide compound of the present invention or the nucleic acid of the present invention or vector of the present invention or host cell of the present invention for the manufacture of a medicament, such as an immunotherapeutic composition or vaccine, for the treatment or prevention of a cancer characterized by expression of human MCP-1 or the MCP-1(1-23) peptide (SEQ ID NO 1).

In a still further aspect, the present invention relates to a peptide compound of the present invention or the nucleic acid of the present invention or vector of the present invention or host cell of the present invention, for use in a method for treatment or prevention of a ca-cer, when administered simultaneously or sequentially with an additional cancer therapy, such as a cytokine therapy, a T-cell therapy, an NK therapy, an immune system checkpoint inhibitor, chemotherapy, radiotherapy, immunostimulating substances, gene therapy, anti-bodies and dendritic cells.

In an embodiment the peptide fragment, nucleic acid, vector or host cell of the present invention wherein the checkpoint blocking antibodies are selected from Actimide, Azacitidine, Azathioprine, Bleomycin, Carboplatin, Capecitabine, Cisplatin, Chlorambucil, Cyclophosphamide, Cytarabine, Dauno-rubicin, Docetaxel, Doxifluridine, Doxorubicin, Epirubicin, Etoposide, Fludarabine, Fluor-ouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Nivolumab, Oxaliplatin, Paclitaxel, Pembrolizumab, Pemetrexed, Revlimid, Temozolomide, Teniposide, Thioguanine, Valrubicin, Vinblastine, Vincristine, Vindesine and Vinorelbine.

Further objects and advantages of the present invention will appear from the following description, and claims.

### Detailed Description of the invention

In the present study, described in more detail in the experimental section, activated T cells are used to target CCL2-expressing cells as a novel method of altering the microenvironment and increasing anti-cancer immunity. CCL2 epitopes could easily be added to ongoing immunotherapeutic measures as a means of strengthening anti-cancer immune responses in patients.

The present findings demonstrate that it was possible for specific T cells to target CCL2-expressing cells. Scrutiny of the CCL2 signal sequence for possible HLA-A2-binding peptides revealed the most promising epitope sequences. The ELISPOT assay described herein was used to examine peripheral blood mononuclear cells (PBMCs) from HLA-A2+ cancer patients and healthy individuals for reactivity against the CCL2-derived T-cell epitopes, and reviled that T cells spontaneously reacted to the CCL2-derived peptides.

In one aspect, the present invention concerns a peptide compound of MCP-1 selected from:
a) a peptide fragment of MKVSAALLCLLLIAATFIPQGLA (MCP-1(1-23)) (SEQ ID NO 1) consisting of a consecutive sequence of from 6 to 22 amino acids,
b) a functional homologue having at least 70%, 80%, 90%, or 95% identity to SEQ ID NO 1 or the peptide fragment of a), and
c) a functional analogue wherein at least one amino acid has been deleted, inserted and/or substituted in SEQ ID NO 1 or the peptide fragment of a),
and wherein the C-terminal amino acid of any one of a), b) or c) also comprises the amide; or a pharmaceutically acceptable salt thereof.

In an embodiment, the peptide compound is selected from b) a functional homologue having at least 70%, 80%, 90%, or 95% identity to SEQ ID NO 1 or the peptide fragment of a), wherein the C-terminal amino acid also comprises the amide; or a pharmaceutically acceptable salt thereof. In one embodiment, the functional homologue has at least 80% identity to SEQ ID NO 1. In a further embodiment, the functional homologue has at least 90% identity to SEQ ID NO 1. In a further embodiment, the functional homologue has at least 95% identity to SEQ ID NO 1. In a further embodiment, the functional homologue has at least 70% identity to the peptide fragment of a). In a further embodiment, the functional homologue has at least 80% identity to the peptide fragment of a). In a further embodiment, the functional homologue has at least 90% identity to the peptide fragment of a). In a further embodiment, the functional homologue has at least 95% identity to the peptide fragment of a).

In another embodiment, the peptide compound is selected from c) a functional analogue wherein at least one amino acid has been deleted, inserted and/or substituted in SEQ ID NO 1 or the peptide fragment of a), wherein the C-terminal amino acid also comprises the amide; or a pharmaceutically acceptable salt thereof.

In a further embodiment, the peptide compound is selected from a) a peptide fragment of SEQ ID NO 1 consisting of a consecutive sequence of from 6 to 22 amino acids, wherein the C-terminal amino acid also comprises the amide;
or a pharmaceutically acceptable salt thereof. In one embodiment, the C-terminal is the acid. In another embodiment, the C-terminal is the amide.

In a further embodiment, the peptide fragment consists of a consecutive sequence in the range of from 8 to 20 amino acids. In a still further embodiment, the peptide fragment consists of a consecutive sequence in the range of from 8 to 16 amino acids. In a further embodiment, the peptide fragment consists of a consecutive sequence in the range of from 8 to 12 amino acids. In a still further embodiment, the peptide fragment consists of a consecutive sequence in the range of from 9-12 amino acids. In a further embodiment, the peptide fragment consists of a consecutive sequence in the range of from 10-14 amino acids.

In a further embodiment, the peptide fragment is selected from the group consisting of MCP-1(1-18), MCP-1(5-18), MCP-1(6-18), MCP-1(7-18), MCP-1(9-18), MCP-1(5-23), MCP-1(6-23), MCP-1(7-23), MCP-1(9-23), and MCP-1(10-23).

In a further embodiment, the consecutive sequence comprises a sequence selected from the group consisting of CLLLIAATF, ALLCLLLIA, LLCLLLIAA, LLLIAATFI, and AALLCLLLI. In one embodiment, the sequence is CLLLIAATF. In another embodiment, the sequence is ALLCLLLIA. In a further embodiment, the sequence is LLCLLLIAA. In a further embodiment, the sequence is LLLIAATFI. In a further embodiment, the sequence is AALLCLLLI.

In a still further embodiment, the peptide fragment under a), the functional homologue under b), or the functional analogue under c) is capable of activating T-cells, such as CD4 and CD8 T-cells. Typically, the activation is determined by the ELISPOT assay described herein.

In a further embodiment, the peptide compound is selected from the group consisting of CLLLIAATF, ALLCLLLIA, LLCLLLIAA, LLLIAATFI, and AALLCLLLI.

It is to be understood that when the peptide fragment consists of a consecutive sequence in the range of from 8 to 20, it may at the same time be selected within the sequence of for instance MCP-1(2-23), whereas a peptide fragment consisting of a consecutive sequence in the range of from 6 to 22, cannot be at the same time be selected within the sequence of for instance MCP-1(7-15), this is known to the person skilled in the art. Otherwise all combinations are contemplated within the present invention.

It is also to be understood that MCP-1(x-y), wherein x and y are integers selected from 1-23 as used herein means a peptide fragment of human peptide MCP-1 having the SEQ ID NO 1 as defined herein (SEQ ID NO 1 is the signal peptide of human MCP-1), wherein x is the N-terminal amino acid and y is the C-terminal amino acid, for instance MCP-1(7-15) indicates the peptide fragment from amino acid 7 of SEQ ID NO 1 to amino acid 15 of SEQ ID NO 1 wherein amino acid 7 is L and amino acid 15 is A.

In a further aspect the present invention concerns a peptide compound of MCP-1 selected from:
a) MCP-1(5-18) or a peptide fragment of AALLCLLLIAATFI (MCP-1(5-18)) (SEQ ID NO 7) consisting of a consecutive sequence of from 6 to 13 amino acids,
b) a functional homologue having at least 70%, 80%, 90%, or 95% identity to SEQ ID NO 7 or the peptide fragment of a), and
c) a functional analogue wherein at least one amino acid has been deleted, inserted and/or substituted in SEQ ID NO 7 or the peptide fragment of a),
and wherein the C-terminal amino acid of any one of a), b) or c) also comprises the amide; or a pharmaceutically acceptable salt thereof. In one embodiment the peptide compound of MCP-1 is selected from a), wherein the C-terminal amino acid of a) also comprises the amide; or a pharmaceutically acceptable salt thereof. In a further embodiment the consecutive sequence has from 8 to 13 amino acids, such as 9 to 12 amino acids. In another embodiment the peptide compound of MCP-1 is AALLCLLLIAATFI (MCP-1(5-18)) (SEQ ID NO 7).

The term "identity" as used herein refers to a relationship between the sequences of two or more peptides, such as polypeptides, as determined by comparing the sequences. In the art, "identity" also means the degree of sequence relatedness between proteins or polypeptides, as determined by the number of matches between strings of two or more amino acid residues. "Identity" measures the percent of identical matches between the smaller of two or more sequences with gap alignments (if any) addressed by a particular mathematical model or computer program (i.e., "algorithms"). Identity of related proteins or peptides can be readily calculated by known methods. Such methods include, but are not limited to, those described in Computational Molecular Biology, Lesk, A. M., ed., Oxford University Press, New York, 1988; Biocomputing: Informatics and Genome Pro-jects, Smith, D. W., ed., Academic Press, New York, 1993; Computer Analysis of Sequence Data, Part 1, Griffin, A. M., and Griffin, H. G., eds., Humana Press, New Jersey, 1994; Se-quence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math., 48, 1073, (1988).

Preferred methods to determine identity are designed to give the largest match between the sequences tested. Methods to determine identity are described in publicly available computer programs. Preferred computer program methods to determine identity between two sequences include the GCG program package, including GAP (Devereux et al., Nucl. Acid. Res., 12, 387, (1984); Genetics Computer Group, University of Wisconsin, Madison, Wis.), BLASTP, BLASTN, and FASTA (Altschul et al., J. Mol. Biol., 215, 403-410, (1990)). The BLASTX program is publicly available from the National Center for Biotechnology Information (NCBI) and other sources (BLAST Manual, Altschul et al. NCB/NLM/NIH Bethesda, Md. 20894; Altschul et al., supra). The well-known Smith Waterman algorithm may also be used to determine identity.

For example, using the computer algorithm GAP (Genetics Computer Group, University of Wisconsin, Madison, Wis.), two proteins for which the percent sequence identity is to be determined are aligned for optimal matching of their respective amino acids (the "matched span", as determined by the algorithm). A gap opening penalty (which is calculated as 3 times the average diagonal; the "average diagonal" is the average of the diagonal of the comparison matrix being used; the "diagonal" is the score or number assigned to each perfect amino acid match by the particular comparison matrix) and a gap extension penalty (which is usually {fraction (1/10)} times the gap opening penalty), as well as a comparison matrix such as PAM 250 or BLOSUM 62 are used in conjunction with the algorithm. A standard comparison matrix (see Dayhoff et al., Atlas of Protein Sequence and Structure, vol. 5, supp.3 (1978) for the PAM 250 comparison matrix; Henikoff et al., Proc. Natl. Acad. Sci USA, 89, 10915-10919, (1992) for the BLOSUM 62 comparison matrix) is also used by the algorithm. Preferred parameters for a protein or peptide sequence comparison include the following: Algorithm: Needleman et al., J. Mol. Biol, 48, 443-453, (1970); Comparison matrix: BLOSUM 62 from Henikoff et al., Proc. Natl. Acad. Sci. USA, 89, 10915-10919, (1992); Gap Penalty: 12, Gap Length Penalty: 4, Threshold of Similarity: 0. The GAP program is useful with the above parameters. The aforementioned parameters are the default parameters for protein comparisons (along with no penalty for end gaps) using the GAP algorithm.

In a further embodiment, the peptide fragment under a) is capable of activating T-cells as determined by the ELISPOT assay described herein.

In a further aspect, the present invention relates to a nucleic acid encoding the peptide compound of the present invention. The peptide compound of the present invention is selected from any one of the above embodiments. In one embodiment, the nucleic acid is selected from the group consisting of DNA and RNA.

In a still further aspect the present invention relates to a vector comprising the nucleic acid of the present invention. The nucleic acid of the present invention is selected from any one of the above embodiments, and the peptide compound of the present invention is selected from any one of the above embodiments. In one embodiment, the vector is selected from a virus vector.

In a further aspect, the present invention relates to a host cell comprising the vector of the present invention. The vector of the present invention is selected from any one of the above embodiments, the nucleic acid of the present invention is selected from any one of the above embodiments, and the peptide compound of the present invention is selected from any one of the above embodiments. In one embodiment, the host cell is selected from a mammalian cell.

In a still further aspect the present invention relates to a composition comprising the MCP-1(1-23) peptide (SEQ ID NO 1) or the peptide compound of the present invention or the nucleic acid of the present invention or the vector of the present invention or the host cell of the present invention, optionally together with a pharmaceutically acceptable additive, such as a carrier or adjuvans.

In a further aspect, the present invention relates to an immunotherapeutic composition comprising
a) the MCP-1 (1-23) peptide (SEQ ID NO 1) or the peptide compound of the present invention or the nucleic acid of the present invention or the vector of the present invention or the host cell of the present invention; and
b) an adjuvant;
for use as a medicament.

In an embodiment, the immunotherapeutic composition of the present invention is for use in a method for treatment or prevention of a disease, disorder or condition selected from cancer. In one embodiment, the cancer is a tumor forming cancer disease. In a further embodiment, the adjuvant is selected from the group consisting of bacterial DNA based adjuvants, oil/surfactant based adjuvants, viral dsRNA based adjuvants, imidazochinilines, and a Montanide ISA adjuvant.

In a further aspect, the present invention relates to a kit-of-parts comprising;
a) the immunotherapeutic composition of the present invention, and
b) a composition comprising at least one second active ingredient, selected from an immunostimulating compound, such as an interleukin, e.g. IL-2 and or IL-21, an anti-cancer
agent, such as a chemotherapeutic agent, e.g. Actimide, Azacitidine, Azathio-prine, Bleomycin, Carboplatin, Capecitabine, Cisplatin, Chlorambucil, Cyclophospha-mide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotec-an, Lenalidomide, Leucovorin, Mechlorethamine, Melphalan, Mercaptopurine, Metho-trexate, Mitoxantrone, nivolumab, Oxaliplatin, Paclitaxel, pembrolizumab, Pemetrexed, Revlimid, Temozolomide, Teniposide, Thioguanine, Valrubicin, Vinblastine, Vincristine, Vindesine and Vinorelbine.

In an embodiment of the kits-of-parts, the provided compositions are to be administered simultaneously or sequentially.

In a further aspect, the present invention relates to a method of treating a clinical condition characterized by expression of human MCP-1, the method comprising administering to an individual suffering from said clinical condition an effective amount of the peptide compound of the present invention or the nucleic acid of the present invention or the vector of the present invention or the host cell of the present invention.

In a still further aspect the present invention relates to use of the peptide compound of the present invention, or the nucleic acid of the present invention, or the vector of the present invention, or the host cell of the present invention, for the manufacture of a medicament, such as an immunotherapeutic composition or vaccine, for the treatment or prevention of a cancer characterized by expression of human MCP-1.

In a further aspect, the present invention relates to the peptide compound of the present invention, or the nucleic acid of the present invention, or the vector of the present invention, or the host cell of the present invention, for use in a method for treatment or prevention of a cancer, when administered simultaneously or sequentially with an additional cancer therapy.

In an embodiment, the additional cancer therapy is selected from the group consisting of a cytokine therapy, a T-cell therapy, an NK therapy, an immune system checkpoint inhibitor, chemotherapy, radiotherapy, immunostimulating substances, gene therapy, anti-bodies and dendritic cells. In one embodiment, the additional cancer therapy is selected from an immune system checkpoint inhibitor. In a particular embodiment, the immune system checkpoint inhibitor is a checkpoint blocking antibody. In a further embodiment the checkpoint blocking antibody is selected from the group consisting of Actimide, Azacitidine, Azathioprine, Bleomycin, Carboplatin, Capecitabine, Cisplatin, Chlorambucil, Cyclophosphamide, Cytarabine, Dauno-rubicin, Docetaxel, Doxifluridine, Doxorubicin, Epirubicin, Etoposide, Fludarabine, Fluor-ouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Nivolumab, Oxaliplatin, Paclitaxel, Pembrolizumab, Pemetrexed, Revlimid, Temozolomide, Teniposide, Thioguanine, Valrubicin, Vinblastine, Vincristine, Vindesine and Vinorelbine.

As used herein any amino acid sequence shown may be modified at the C-terminal amino acid to be on amide form (-CONH₂) or may be on acid form (-COOH), thus any one of these are preferred embodiments, and it is intended that any C-terminal amino acid, such as I, L, V, comprises both amide and acid form unless specified by -NH₂ or -OH.

The MCP-1 peptide fragments disclosed herein are made by standard peptide synthesis, such as solid-phase peptide synthesis (SPPS). SPPS is a standard method for synthesizing peptides in the lab. SPPS allows for the synthesis of natural peptides which are difficult to express in bacteria, the incorporation of unnatural amino acids, peptide/protein backbone modification, and the synthesis of D-proteins, which consist of D-amino acids. Small porous beads are treated with functional units ('linkers') on which peptide chains can be built. The peptide will remain covalently attached to the bead until cleaved from it by a reagent such as anhydrous hydrogen fluoride or trifluoroacetic acid. The peptide is thus 'immobilized' on the solid-phase and can be retained during a filtration process while liquid-phase reagents and by-products of synthesis are flushed away. The general principle of SPPS is one of repeated cycles of deprotection-wash-coupling-wash. The free N-terminal amine of a solid-phase attached peptide is coupled to a single N-protected amino acid unit. This unit is then deprotected, revealing a new N-terminal amine to which a further amino acid may be attached. The superiority of this technique partially lies in the ability to perform wash cycles after each reaction, removing excess reagent with all of the growing peptide of interest remaining covalently attached to the insoluble resin. There are two majorly used forms of SPPS - Fmoc and Boc. Unlike ribosome protein synthesis, solid-phase peptide synthesis proceeds in a C-terminal to N-terminal fashion. The N-termini of amino acid monomers is protected by either of these two groups and added onto a deprotected amino acid chain. Automated synthesizers are available for both techniques, though many research groups continue to perform SPPS manually. Furthermore, the skilled person will understand that the processes described above and hereinafter the functional groups of intermediate compounds may need to be protected by protecting group.

When the peptide compounds, nucleic acids, vectors, host cells and pharmaceutical compositions herein disclosed are used for the above treatment, a therapeutically effective amount of at least one compound is administered to a mammal in need of said treatment.

As used herein amino acids are identified by the one or three letter code known to the person skilled in the art and shown in the table below for convenience:

**Amino acids, one and three letter codes**

| **Amino acid** | **Three letter code** | **One letter code** |
|---|---|---|
| alanine | ala | A |
| arginine | arg | R |
| asparagine | asn | N |
| aspartic acid | asp | D |
| asparagine or aspartic acid | asx | B |
| cysteine | cys | C |
| glutamic acid | glu | E |
| glutamine | gln | Q |
| glutamine or glutamic acid | glx | z |
| glycine | gly | G |
| histidine | his | H |
| isoleucine | ile | I |
| leucine | leu | L |
| lysine | lys | K |
| methionine | met | M |
| phenylalanine | phe | F |
| proline | pro | P |
| serine | ser | S |
| threonine | thr | T |
| tryptophan | trp | W |
| tyrosine | tyr | Y |
| valine | val | V |

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. The treatment may either be performed in an acute or in a chronic way. The patient to be treated is preferably a mammal; in particular a human being, but it may also include animals, such as dogs, cats, cows, monkeys, apes, sheep and pigs.

The term "a therapeutically effective amount" of a peptide compound of the present invention or a peptide fragment disclosed herein, as used herein means an amount sufficient to cure, alleviate or partially arrest the clinical manifestations of a given disease and its complications. An amount adequate to accomplish this is defined as "therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician or veterinary.

In a still further aspect the present invention relates to a pharmaceutical composition comprising the peptide compound, such as peptide fragment, of the present invention and optionally a pharmaceutically acceptable additive, such as a carrier or an excipient.

As used herein "pharmaceutically acceptable additive" is intended without limitation to include carriers, excipients, diluents, adjuvant, colorings, aroma, preservatives etc. that the skilled person would consider using when formulating a compound of the present invention in order to make a pharmaceutical composition.

The adjuvants, diluents, excipients and/or carriers that may be used in the composition of the invention must be pharmaceutically acceptable in the sense of being compatible with the peptide compound, peptide fragment, nucleic acid, vector, or host cell and the other ingredients of the pharmaceutical composition, and not deleterious to the recipient thereof. It is preferred that the compositions shall not contain any material that may cause an adverse reaction, such as an allergic reaction. The adjuvants, diluents, excipients and carriers that may be used in the pharmaceutical composition of the invention are well known to a person within the art.

Adjuvants are any substance whose admixture into the composition increases or otherwise modifies the immune response elicited by the composition. Adjuvants, broadly defined, are substances which promote immune responses. Adjuvants may also preferably have a depot effect, in that they also result in a slow and sustained release of an active agent from the administration site. A general discussion of adjuvants is provided in Goding, Monoclonal Antibodies: Principles & Practice (2nd edition, 1986) at pages 61-63.

Adjuvants may be selected from the group consisting of: AlK(SO4)2, AlNa(SO4)2, AlNH4 (SO4), silica, alum, Al(OH)3, Ca3 (PO4)2, kaolin, carbon, aluminum hydroxide, muramyl dipeptides, N-acetyl-muramyl-L-threonyl-D-isoglutamine (thr-DMP), N-acetyl-nornuramyl-L-alanyl-D-isoglutamine (CGP 11687, also referred to as nor-MDP), N-acetylmuramyul-L-alanyl-D-isoglutaminyl-L-alanine-2-(1'2'-dipalmitoyl-sn -glycero-3-hydroxphosphoryloxy)-ethylamine (CGP 19835A, also referred to as MTP-PE), RIBI (MPL+TDM+CWS) in a 2% squalene/Tween-80.RTM. emulsion, lipopolysaccharides and its various derivatives, including lipid A, Freund's Complete Adjuvant (FCA), Freund's Incomplete Adjuvants, Merck Adjuvant 65, polynucleotides (for example, poly IC and poly AU acids), wax D from Mycobacterium, tuberculosis, substances found in Corynebacterium parvum, Bordetella pertussis, and members of the genus Brucella, Titermax, ISCOMS, Quil A, ALUN (see US 58767 and 5,554,372), Lipid A derivatives, choleratoxin derivatives, HSP derivatives, LPS derivatives, synthetic peptide matrixes or GMDP, Interleukin 1, Interleukin 2, Montanide ISA-51 and QS-21. Various saponin extracts have also been suggested to be useful as adjuvants in immunogenic compositions. Granulocyte-macrophage colony stimulating factor (GM-CSF) may also be used as an adjuvant.

Preferred adjuvants to be used with the invention include oil/surfactant based adjuvants such as Montanide adjuvants (available from Seppic, Belgium), preferably Montanide ISA-51. Other preferred adjuvants are bacterial DNA based adjuvants, such as adjuvants including CpG oligonucleotide sequences. Yet other preferred adjuvants are viral dsRNA based adjuvants, such as poly I:C. GM-CSF and Imidazochinilines are also examples of preferred adjuvants.

The adjuvant is most preferably a Montanide ISA adjuvant. The Montanide ISA adjuvant is preferably Montanide ISA 51 or Montanide ISA 720.

In Goding, Monoclonal Antibodies: Principles & Practice (2nd edition, 1986) at pages 61-63 it is also noted that, when an antigen of interest is of low molecular weight, or is poorly immunogenic, coupling to an immunogenic carrier is recommended. A peptide compound, peptide fragment, nucleic acid, vector, or host cell of an immunotherapeutic composition of the invention may be coupled to a carrier. A carrier may be present independently of an adjuvant. The function of a carrier can be, for example, to increase the molecular weight of the peptide compound, peptide fragment, nucleic acid, vector, or host cell in order to increase activity or immunogenicity, to confer stability, to increase the biological activity, or to increase serum half-life. Furthermore, a carrier may aid in presenting the polypeptide or fragment thereof to T-cells. Thus, in the immunogenic composition, the polypeptide or fragment thereof may be associated with a carrier, such as those set out below.

The carrier may be any suitable carrier known to a person skilled in the art, for example a protein or an antigen presenting cell, such as a dendritic cell (DC). Carrier proteins include keyhole limpet hemocyanin, serum proteins such as transferrin, bovine serum albumin, human serum albumin, thyroglobulin or ovalbumin, immunoglobulins, or hormones, such as insulin or palmitic acid. Alternatively, the carrier protein may be tetanus toxoid or diphtheria toxoid. Alternatively, the carrier may be a dextran such as sepharose. The carrier must be physiologically acceptable to humans and safe.

The immunotherapeutic composition may optionally comprise a pharmaceutically acceptable excipient. The excipient must be 'acceptable' in the sense of being compatible with the other ingredients of the composition and not deleterious to the recipient thereof. Auxiliary substances, such as wetting or emulsifying agents, pH buffering substances and the like, may be present in the excipient. These excipients and auxiliary substances are generally pharmaceutical agents that do not induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients, vehicles and auxiliary substances is available in Remington's Pharmaceutical Sciences (Mack Pub. Co., N.J. 1991).

The immunotherapeutic composition may be prepared, packaged, or sold in a form suitable for bolus administration or for continuous administration. Injectable compositions may be prepared, packaged, or sold in unit dosage form, such as in ampoules or in multi-dose containers containing a preservative. Compositions include, but are not limited to, suspensions, solutions, emulsions in oily or aqueous vehicles, pastes, and implantable sustained-release or biodegradable formulations. In one embodiment of a composition, the active ingredient is provided in dry (for e.g., a powder or granules) form for reconstitution with a suitable vehicle (e. g., sterile pyrogen-free water) prior to administration of the reconstituted composition. The composition may be prepared, packaged, or sold in the form of a sterile injectable aqueous or oily suspension or solution. This suspension or solution may be formulated according to the known art, and may comprise, in addition to the active ingredient, additional ingredients such as the adjuvants, excipients and auxiliary substances described herein. Such sterile injectable formulations may be prepared using a non-toxic parenterally-acceptable diluent or solvent, such as water or 1,3-butane diol, for example. Other acceptable diluents and solvents include, but are not limited to, Ringer's solution, isotonic sodium chloride solution, and fixed oils such as synthetic mono- or di-glycerides.

Other compositions which are useful include those which comprise the active ingredient in microcrystalline form, in a liposomal preparation, or as a component of a biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. Alternatively, the active ingredients of the composition may be encapsulated, adsorbed to, or associated with, particulate carriers. Suitable particulate carriers include those derived from polymethyl methacrylate polymers, as well as PLG microparticles derived from poly(lactides) and poly(lactide-co-glycolides). See, e.g., Jeffery et al. (1993) Pharm. Res. 10:362-368. Other particulate systems and polymers can also be used, for example, polymers such as polylysine, polyarginine, polyomithine, spermine, spermidine, as well as conjugates of these molecules.

As mentioned above, the compositions and particularly immunotherapetic compositions as herein disclosed may, in addition to the compounds herein disclosed, further comprise at least one pharmaceutically acceptable adjuvant, diluent, excipient and/or carrier. In some embodiments, the pharmaceutical compositions comprise from 1 to 99 weight % of said at least one pharmaceutically acceptable adjuvant, diluent, excipient and/or carrier and from 1 to 99 weight % of a compound as herein disclosed. The combined amount of the active ingredient and of the pharmaceutically acceptable adjuvant, diluent, excipient and/or carrier may not constitute more than 100% by weight of the composition, particularly the pharmaceutical composition.

In some embodiments, only one compound as herein disclosed is used for the purposes discussed above.

In some embodiments, two or more of the compound as herein disclosed are used in combination for the purposes discussed above.

The composition, particularly immunotherapeutic composition comprising a compound set forth herein may be adapted for oral, intravenous, topical, intraperitoneal, nasal, buccal, sublingual, or subcutaneous administration, or for administration via the respiratory tract in the form of, for example, an aerosol or an air-suspended fine powder. Therefore, the pharmaceutical composition may be in the form of, for example, tablets, capsules, powders, nanoparticles, crystals, amorphous substances, solutions, transdermal patches or suppositories.

Further embodiments of the process are described in the experimental section herein, and each individual process as well as each starting material constitutes embodiments that may form part of embodiments.

The above embodiments should be seen as referring to any one of the aspects (such as 'method for treatment', 'immunotherapeutic composition', 'peptide compound for use as a medicament', or 'peptide compound for use in a method') described herein as well as any one of the embodiments described herein unless it is specified that an embodiment relates to a certain aspect or aspects of the present invention.

All references, including publications, patent applications and patents, cited herein are hereby incorporated by reference to the same extent as if each reference was individually and specifically indicated to be incorporated by reference and was set forth in its entirety herein.

All headings and sub-headings are used herein for convenience only and should not be construed as limiting the invention in any way.

Any combination of the above-described elements in all possible variations thereof is encompassed by the invention unless otherwise indicated herein or otherwise clearly contradicted by context.

The terms "a" and "an" and "the" and similar referents as used in the context of describing the invention are to be construed to cover both the singular and the plural, unless otherwise indicated herein or clearly contradicted by context.

Recitation of ranges of values herein are merely intended to serve as a shorthand method of referring individually to each separate value falling within the range, unless otherwise indicated herein, and each separate value is incorporated into the specification as if it were individually recited herein. Unless otherwise stated, all exact values provided herein are representative of corresponding approximate values (e.g., all exact exemplary values provided with respect to a particular factor or measurement can be considered to also pro-vide a corresponding approximate measurement, modified by "about," where appropriate).

All methods described herein can be performed in any suitable order unless otherwise indicated herein or otherwise clearly contradicted by context.

The use of any and all examples, or exemplary language (*e.g*., "such as") provided herein, is intended merely to better illuminate the invention and does not pose a limitation on the scope of the invention unless otherwise indicated. No language in the specification should be construed as indicating any element is essential to the practice of the invention unless as much is explicitly stated.

The citation and incorporation of patent documents herein is done for convenience only and does not reflect any view of the validity, patentability and/or enforceability of such patent documents.

The description herein of any aspect or embodiment of the invention using terms such as "comprising", "having", "including" or "containing" with reference to an element or elements is intended to provide support for a similar aspect or embodiment of the invention that "consists of', "consists essentially of', or "substantially comprises" that particular element or elements, unless otherwise stated or clearly contradicted by context (*e.g.*, a composition described herein as comprising a particular element should be understood as also describing a composition consisting of that element, unless otherwise stated or clearly contradicted by context).

This invention includes all modifications and equivalents of the subject matter recited in the aspects or claims presented herein to the maximum extent permitted by applicable law.

The present invention is further illustrated by the following examples that, however, are not to be construed as limiting the scope of protection. The features disclosed in the foregoing description and in the following examples may, both separately and in any combination thereof, be material for realizing the invention in diverse forms thereof.

### Experimental

### Patients, protocols and methods

### Patient material:

The present project is based on analyses of blood and tumor lesions from melanoma, prostate and breast cancer patients. Material from cancer patients is collected at Herlev Hospital. The immediate processing of the material is handled at the CCIT. CCIT are currently running several clinical vaccination trials (www.clinicaltrial.gov) and research projects based on studies of patient samples that are approved by the local ethics committee. Hence, it should be noted that all patients only participate upon written informed consent, and that the ethical committee have approved the projects.

### Enzyme-Linked Immunospot (ELISPOT):

PBMCs from healthy donors or cancer patients were stimulated with 80µg of CCL2-derived peptides and 120 U/ml IL-2 (Peprotech, London, UK, cat. 200-02) for a week. 4-6x10⁵ PBMCs were then placed in the bottom of ELISPOT plate (nitrocellulose bottomed 96-well plates by MultiScreen MAIP N45; Millipore, cat. MSIPN4W50) pre-coated with IFN-γ capture Ab (Mabtech, cat. 3420-3-1000) and 1-10µg of CCL2 derived peptides were added. PBMCs from each patient and donors were set up in duplicates or triplicates for peptide and control stimulations. Cells were incubated in ELISPOT plates in the presence of an antigen for 14-16 hours after which they are washed off and secondary biotinylated Ab (Mabtech, cat. 3420-6-1000) was added. After 2h incubation unbound secondary antibody was washed off and streptavidin conjugated alkaline phosphatase (AP) (Mabtech, cat. 3310-10) was added for 1 h. Next, unbound conjugated enzyme is washed off and the assay is developed by adding BCIP/NBT substrate (Mabtech, cat. 3650-10). Developed ELISPOT plates were analysed on CTL ImmunoSpot S6 Ultimate-V analyzer using Immunospot software v5.1.

### Cytotoxicity assay

Conventional ⁵¹Cr-release assays for CTL-mediated cytotoxicity were carried out as described elsewhere (reference 23) Target cells were T2-cells (ATCC). T2-cells were pulsed with peptides during labeling with chrome.

American Type Culture Collection provided the cell line T2. All cell lines included in the study were and tested and authenticated by HLA genotyping. The cell lines were routinely confirmed with their HLA typing and antigen expression by flow cytometry and coculture assays, respectively.

### Results

### Spontaneous Immunity towards CCL2

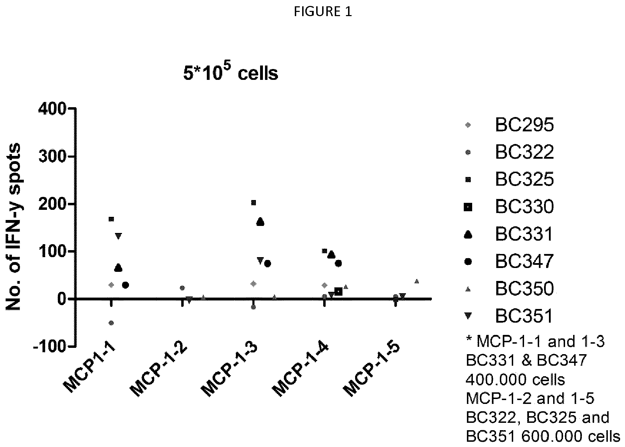

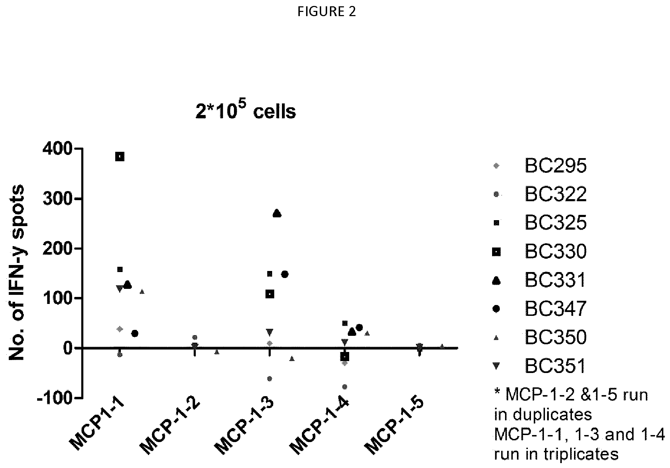

The amino acid sequence of the signal peptide of the MCP-1 protein was screened with the "SYFPEITHI" database to predict the best HLA-A2 peptide epitopes. The algorithm identified 5 peptides that were top candidates. We selected the 5 peptides for synthesis and further study. These 5 peptides were used with the IFN-γ ELISPOT *in vitro* assay to test for the presence of specific T-cell responses in PBMCs from 8 different HLA-A2⁺ healthy donors; 5x10e5 (figure 1) or 2x10e5 (figure2). We detected immune responses against MCP1-1 (SEQ ID NO 2), MCP1-3 (SEQ ID NO 4), MCP-1-4 (SEQ ID NO 5) and MCP1-5 (SEQ ID NO 6). In particular, against MCP1-1, MCP1-3, and MCP-1-4.

Figure 1 shows *In-vitro* IFN-γ ELISPOT results. PBMCs from 8 healthy donors stimulated once *in vitro* with each peptide. Then, the PBMCs were exposed to the peptides, and IFN-γ secretion was measured with ELISPOT. The response was calculated as the number of peptide-specific spots, minus the number of spots that reacted to an irrelevant peptide (HIV/HLA-A2; pol476-484; ILKEPVHGV) per 5×10⁵ PBMCs. Screening in 8xHD (MCP-1-2 (SEQ ID NO 3) and 1-5 only in 4xHD -> BC322, 25, 50 and 51). Samples run in triplicates (MCP-1-2 and 1-5 in duplicates) and data presented as mean. Missing samples = TNTC

Figure 2 shows *In-vitro* IFN-γ ELISPOT results. PBMCs from 8 healthy donors stimulated once *in vitro* with each peptide. Then, the PBMCs were exposed to the peptides, and IFN-γ secretion was measured with ELISPOT. The response was calculated as the number of peptide-specific spots, minus the number of spots that reacted to an irrelevant peptide (HIV/HLA-A2; pol476-484; ILKEPVHGV) per 2×10⁵ PBMCs. Screening in 8xHD (MCP-1-2 and 1-5 only in 4xHD -> BC322, 25, 50 and 51). Samples run in triplicates (MCP-1-2 and 1-5 in duplicates) and data presented as mean. Missing samples = TNTC

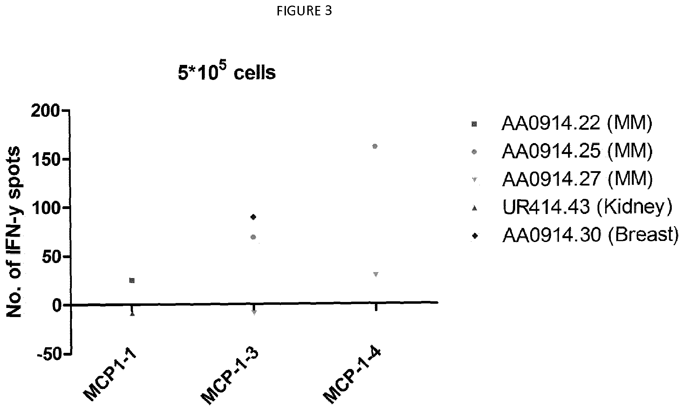

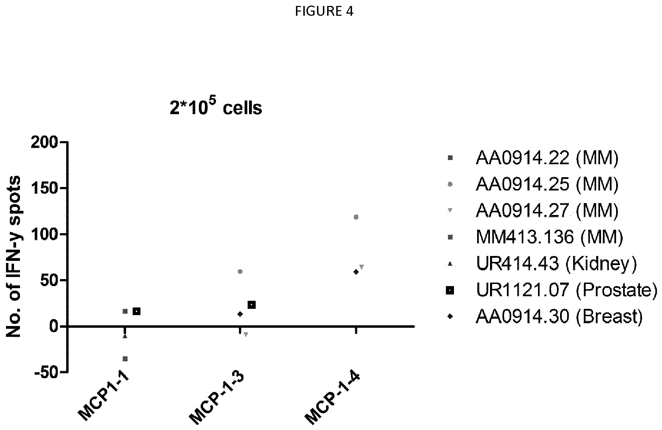

Next, we tested PBMCs from cancer patients for immune responses against MCP1-1, MCP1-3, and MCP-1-4 with the IFN-y ELISPOT assay in PMBC from seven cancer patients (4 patients with malignant melanoma (MM), 1 kidney cancer patient, 1 prostate cancer patient and 1 breast cancer patient). We detected strong immune responses against MCP1-4 and MCP1-3, and weaker responses against MCP1-1 (Figure 3 and 4).

Figure 3 shows *In-vitro* IFN-γ ELISPOT results. PBMCs from 7 patients with cancer stimulated once *in vitro* with each peptide. Then, the PBMCs were exposed to the peptides, and IFN-γ secretion was measured with ELISPOT. The response was calculated as the number of peptide-specific spots, minus the number of spots that reacted to an irrelevant peptide (HIV/HLA-A2; pol476-484; ILKEPVHGV) per 5×10⁵ PBMCs. Screening in 4xCancer patients for each peptide, as MCP-1-1 was predicted to be A3 reactive and the others were A2. Samples run in triplicates and data presented as mean. MCP-1-1: UR1121.07, AA914.22, MM413.136 and UR414.43. MCP-1-3 and 1-4: UR1121.07, AA914.25, AA914.27, AA914.30.

Figure 4 shows *In-vitro* IFN-γ ELISPOT results. PBMCs from 7 patients with cancer stimulated once *in vitro* with each peptide. Then, the PBMCs were exposed to the peptides, and IFN-γ secretion was measured with ELISPOT. The response was calculated as the number of peptide-specific spots, minus the number of spots that reacted to an irrelevant peptide (HIV/HLA-A2; pol476-484; ILKEPVHGV) per 2×10⁵ PBMCs. Screening in 4xCancer patients for each peptide, as MCP-1-1 was predicted to be A3 reactive and the others were A2. Samples run in triplicates and data presented as mean. MCP-1-1: UR1121.07, AA914.22, MM413.136 and UR414.43. MCP-1-3 and 1-4: UR1121.07, AA914.25, AA914.27, AA914.30.

### Specific T cells

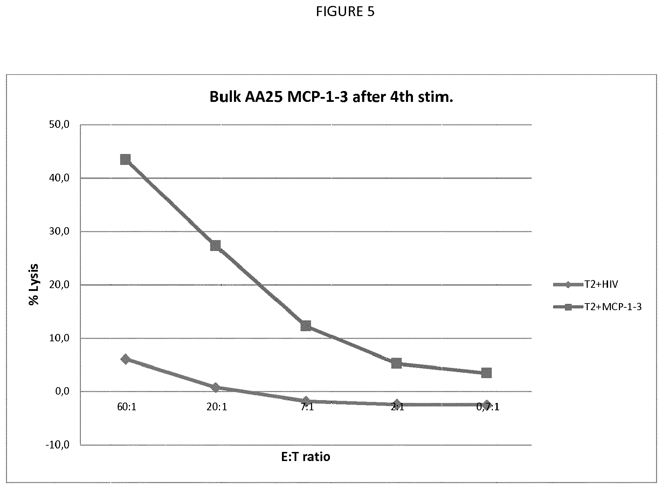

To characterize the immune response elicited by MCP1, we expanded MCP1-3-specific T cells. Briefly, PBMCs isolated from a patient with melanoma were expanded *in vitro.* Then, PBMCs were stimulated with autologous DCs that had been pulsed with MCP1-3. These T cells were cultured and expanded with high dose IL-2, then analyzed for specificity against MCP1-3 in cytotoxicity assays (Figure 5). The T-cells recognized and lysed T2 cells that had been pulsed with MCP1-3 in conventional ⁵¹chromium-release assays, but they did not recognize T2 cells pulsed with an irrelevant HIV peptide (HIV-1 pol476-484) (Figure 5).

Figure 5 shows that **MCP1-3-specific T cells are cytolytic and functional** T2 cells, pulsed with MCP1-3 were lysed by specific T cells, whereas T2 cells pulsed with an irrelevant peptide were not recognized.

**Sequences used in the specification:**
Signal peptide MCP-1(1-23):
   MKVSAALLCLLLIAATFIPQGLA (SEQ ID NO. 1)
MCP-1 (9-17):
   CLLLIAATF (SEQ ID NO. 2)
MCP-1 (6-14):
   ALLCLLLIA (SEQ ID NO. 3)
MCP-1 (7-15):
   LLCLLLIAA (SEQ ID NO. 4)
MCP-1 (10-18):
   LLLIAATFI (SEQ ID NO. 5)
MCP-1 (5-13):
   AALLCLLLI (SEQ ID NO. 6)
MCP-1(5-18):
   AALLCLLLIAATFI (SEQ ID NO 7)

### References

(1) Lazennec G, Richmond A. Chemokines and chemokine receptors: new insights into cancer-related inflammation. Trends Mol Med 2010;16:133-44.
(2) Balkwill FR. The chemokine system and cancer. J Pathol 2012;226:148-57.
(3) Zhang J, Patel L, Pienta KJ. Targeting chemokine (C-C motif) ligand 2 (CCL2) as an example of translation of cancer molecular biology to the clinic. Prog Mol Biol Transl Sci 2010;95:31-53. doi: 10.1016/B978-0-12-385071-3.00003-4.:31-53.
(4) Qian BZ, Li J, Zhang H, Kitamura T, Zhang J, Campion LR, et al. CCL2 recruits inflammatory monocytes to facilitate breast-tumour metastasis. Nature 2011;475:222-5.
(5) Zhang J, Lu Y, Pienta KJ. Multiple roles of chemokine (C-C motif) ligand 2 in promoting prostate cancer growth. J Natl Cancer Inst 2010;102:522-8.
(6) Murdoch C, Giannoudis A, Lewis CE. Mechanisms regulating the recruitment of macrophages into hypoxic areas of tumors and other ischemic tissues. Blood 2004; 104:2224-34.
(7) Li X, Loberg R, Liao J, Ying C, Snyder LA, Pienta KJ, et al. A destructive cascade mediated by CCL2 facilitates prostate cancer growth in bone. Cancer Res 2009;69:1685-92.
(8) Fujimoto H, Sangai T, Ishii G, Ikehara A, Nagashima T, Miyazaki M, et al. Stromal MCP-1 in mammary tumors induces tumor-associated macrophage infiltration and contributes to tumor progression. Int J Cancer 2009;125:1276-84.
(9) Sahin H, Trautwein C, Wasmuth HE. Functional role of chemokines in liver disease models. Nat Rev Gastroenterol Hepatol 2010;7:682-90.
(10) Mizutani K, Sud S, McGregor NA, Martinovski G, Rice BT, Craig MJ, et al. The chemokine CCL2 increases prostate tumor growth and bone metastasis through macrophage and osteoclast recruitment. Neoplasia 2009; 11:1235-42.
(11) Negus RP, Stamp GW, Relf MG, Burke F, Malik ST, Bernasconi S, et al. The detection and localization of monocyte chemoattractant protein-1 (MCP-1) in human ovarian cancer. J Clin Invest 1995;95:2391-6.
(12) Arenberg DA, Keane MP, DiGiovine B, Kunkel SL, Strom SR, Burdick MD, et al. Macrophage infiltration in human non-small-cell lung cancer: the role of CC chemokines. Cancer Immunol Immunother 2000;49:63-70.
(13) Huang B, Lei Z, Zhao J, Gong W, Liu J, Chen Z, et al. CCL2/CCR2 pathway mediates recruitment of myeloid suppressor cells to cancers. Cancer Lett 2007;252:86-92.
(14) Chun E, Lavoie S, Michaud M, Gallini CA, Kim J, Soucy G, et al. CCL2 Promotes Colorectal Carcinogenesis by Enhancing Polymorphonuclear Myeloid-Derived Suppressor Cell Population and Function. Cell Rep 2015;12:244-57.
(15) Roca H, Varsos ZS, Sud S, Craig MJ, Ying C, Pienta KJ. CCL2 and interleukin-6 promote survival of human CD11b+ peripheral blood mononuclear cells and induce M2-type macrophage polarization. J Biol Chem 2009;284:34342-54.
(16) Sierra-Filardi E, Nieto C, Dominguez-Soto A, Barroso R, Sanchez-Mateos P, Puig-Kroger A, et al. CCL2 shapes macrophage polarization by GM-CSF and M-CSF: identification of CCL2/CCR2-dependent gene expression profile. J Immunol 2014;192:3858-67.
(17) Takaishi K, Komohara Y, Tashiro H, Ohtake H, Nakagawa T, Katabuchi H, et al. Involvement of M2-polarized macrophages in the ascites from advanced epithelial ovarian carcinoma in tumor progression via Stat3 activation. Cancer Sci 2010; 101:2128-36.
(18) Sica A, Saccani A, Bottazzi B, Bernasconi S, Allavena P, Gaetano B, et al. Defective expression of the monocyte chemotactic protein-1 receptor CCR2 in macrophages associated with human ovarian carcinoma. J Immunol 2000;164:733-8.
(19) Kawamura K, Komohara Y, Takaishi K, Katabuchi H, Takeya M. Detection of M2 macrophages and colony-stimulating factor 1 expression in serous and mucinous ovarian epithelial tumors. Pathol Int 2009;59:300-5.
(20) Pienta KJ, Machiels JP, Schrijvers D, Alekseev B, Shkolnik M, Crabb SJ, et al. Phase 2 study of carlumab (CNTO 888), a human monoclonal antibody against CC-chemokine ligand 2 (CCL2), in metastatic castration-resistant prostate cancer. Invest New Drugs 2013;31:760-8.
(21) Henderson RA, Michel H, Sakaguchi K, Shabanowitz J, Appella E, Hunt DF, et al. HLA-A2.1-associated peptides from a mutant cell line: a second pathway of antigen presentation. Science 1992;255:1264-6.
(22) Wolfel C, Drexler I, Van Pel A, Thres T, Leister N, Herr W, et al. Transporter (TAP)- and proteasome-independent presentation of a melanoma-associated tyrosinase epitope. Int J Cancer 2000;88:432-8.
(23) Munir S, Andersen GH, Met O, Donia M, Frosig TM, Larsen SK, et al. HLA-restricted cytotoxic T cells that are specific for the immune checkpoint ligand PD-L1 occur with high frequency in cancer patients. Cancer Research 2013;73:1674-776.
(24) Martinenaite E, Ahmad SM, Hansen M, Met O, Westergaard MW, Larsen SK, et al. CCL22-specific T cells: Modulating the Immunosuppressive Tumor Microenvironment. Oncoimmunology 2016;5:e1238541.
(25) Curiel TJ, Coukos G, Zou L, Alvarez X, Cheng P, Mottram P, et al. Specific recruitment of regulatory T cells in ovarian carcinoma fosters immune privilege and predicts reduced survival. Nat Med 2004;10:942-9.
(26) Gobert M, Treilleux I, Bendriss-Vermare N, Bachelot T, Goddard-Leon S, Arfi V, et al. Regulatory T cells recruited through CCL22/CCR4 are selectively activated in lymphoid infiltrates surrounding primary breast tumors and lead to an adverse clinical outcome. Cancer Res 2009;69:2000-9.
(27) Zhou M, Bracci PM, McCoy LS, Hsuang G, Wiemels JL, Rice T, et al. Serum macrophage-derived chemokine/CCL22 levels are associated with glioma risk, CD4 T cell lymphopenia and survival time. Int J Cancer 2015;137:826-36.
(28) Menetrier-Caux C, Faget J, Biota C, Gobert M, Blay JY, Caux C. Innate immune recognition of breast tumor cells mediates CCL22 secretion favoring Treg recruitment within tumor environment. Oncoimmunology 2012;1:759-61.
(29) Cao L, Hu X, Zhang J, Huang G, Zhang Y. The role of the CCL22-CCR4 axis in the metastasis of gastric cancer cells into omental milky spots. J Transl Med 2014; 12:267. doi: 10.1186/s12967-014-0267-1.:267-0267.

## Claims

1. A peptide compound of MCP-1 selected from:
a) a peptide fragment of MKVSAALLCLLLIAATFIPQGLA (MCP-1(1-23)) (SEQ ID NO 1) consisting of a consecutive sequence of from 6 to 22 amino acids,
b) a functional homologue having at least 70%, 80%, 90%, or 95% identity to SEQ ID NO 1 or the peptide fragment of a), and
c) a functional analogue wherein at least one amino acid has been deleted, inserted and/or substituted in SEQ ID NO 1 or the peptide fragment of a),
and wherein the C-terminal amino acid of any one of a), b) or c) also comprises the amide; or a pharmaceutically acceptable salt thereof.

2. The peptide compound of claim 1 selected from a) a peptide fragment of SEQ ID NO 1 consisting of a consecutive sequence of from 6 to 22 amino acids, wherein the C-terminal amino acid also comprises the amide;
or a pharmaceutically acceptable salt thereof.

3. The peptide compound of claim 1 or 2 wherein the peptide fragment consists of a consecutive sequence in the range of from 8 to 20 amino acids, 8 to 16 amino acids, 8 to 12 amino acids, or 9-12 amino acids.

4. The peptide compound of any one of claims 2-3 wherein the peptide fragment of SEQ ID NO 1 is selected from the group consisting of MCP-1 (1-18), MCP-1 (5-18), MCP-1 (6-18), MCP-1(7-18), MCP-1(9-18), MCP-1(5-23), MCP-1(6-23), MCP-1(7-23), MCP-1(9-23), and MCP-1(10-23).

5. A peptide compound of MCP-1 selected from:
a) MCP-1(5-18) or a peptide fragment of AALLCLLLIAATFI (MCP-1(5-18)) (SEQ ID NO 7) consisting of a consecutive sequence of from 6 to 13 amino acids,
b) a functional homologue having at least 70%, 80%, 90%, or 95% identity to SEQ ID NO 7 or the peptide fragment of a), and
c) a functional analogue wherein at least one amino acid has been deleted, inserted and/or substituted in SEQ ID NO 7 or the peptide fragment of a),
and wherein the C-terminal amino acid of any one of a), b) or c) also comprises the amide; or a pharmaceutically acceptable salt thereof.

6. The peptide compound of any one of claims 2-5 wherein the consecutive sequence comprises a sequence selected from the group consisting of CLLLIAATF, ALLCLLLIA, LLCLLLIAA, LLLIAATFI, and AALLCLLLI.

7. The peptide compound of any one of claims 1-6 wherein the peptide fragment under a), the functional homologue under b), or the functional analogue under c) is capable of activating T-cells, such as CD4 and CD8 T-cells.

8. The peptide compound of claim 7 wherein the activation is determined by the ELISPOT assay described herein.

9. The peptide compound of any one of claims 1-8 wherein the peptide compound is selected from the group consisting of
CLLLIAATF,
ALLCLLLIA,
LLCLLLIAA,
LLLIAATFI, and
AALLCLLLI.

10. A nucleic acid, such as DNA or RNA, encoding the peptide compound of any one of the preceding claims.

11. A vector, such virus vector, comprising the nucleic acid of claim 10.

12. A host cell, such as mammalian cell, comprising the vector of claim 11.

13. A composition comprising the MCP-1(1-23) peptide (SEQ ID NO 1) or the peptide compound of any one of claims 1-9 or the nucleic acid of claim 10 or the vector of claim 11 or the host cell of claim 11, optionally together with a pharmaceutically acceptable additive, such as a carrier or adjuvans.

14. An immunotherapeutic composition comprising
a) the MCP-1(1-23) peptide (SEQ ID NO 1) or the peptide compound of any one of claims 1-9 or the nucleic acid of claim 10 or the vector of claim 11 or the host cell of claim 11; and
b) an adjuvant;
for use as a medicament.

15. The immunotherapeutic composition of claim 14 for use in a method for treatment or prevention of a disease, disorder or condition selected from cancer, such as a tumor forming cancer disease.

16. The immunotherapeutic composition of any one of claims 14-15 wherein the adjuvant is selected from the group consisting of bacterial DNA based adjuvants, oil/surfactant based adjuvants, viral dsRNA based adjuvants, imidazochinilines, a Montanide ISA adjuvant.

17. A kit-of-parts comprising;
a) the immunotherapeutic composition of any one of claims 14-16, and
b) a composition comprising at least one second active ingredient, selected from an immunostimulating compound, such as an interleukin, e.g. IL-2 and or IL-21, an anti-cancer agent, such as a chemotherapeutic agent, e.g. Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, nivolumab, Oxaliplatin, Paclitaxel, pembrolizumab, Pemetrexed, Revlimid, Temozolomide, Teniposide, Thioguanine, Valrubicin, Vinblastine, Vincristine, Vindesine and Vinorelbine.

18. The kits-of-parts according to claim 17, where the provided compositions are to be administered simultaneously or sequentially.

19. A method of treating a clinical condition **characterized by** expression of human MCP-1/CCL2 or the MCP-1(1-23) peptide (SEQ ID NO 1), the method comprising administering to an individual suffering from said clinical condition an effective amount of the peptide compound of any one of claims 1-9 or the nucleic acid of claim 10 or vector of claim 11 or host cell of claim 12.

20. A method of treating or preventing cancer in a patient, the method comprising administering to the cancer patient an effective amount of the peptide compound of any one of claims 1-9 or the nucleic acid of claim 10 or vector of claim 11 or host cell of claim 12.

21. Use of the peptide compound of any one of claims 1-9 or the nucleic acid of claim 10 or vector of claim 11 or host cell of claim 12 for the manufacture of a medicament, such as an immunotherapeutic composition or vaccine, for the treatment or prevention of a cancer **characterized by** expression of human MCP-1 or the MCP-1(1-23) peptide (SEQ ID NO 1).

22. A peptide compound of any one of claims 1-9 or the nucleic acid of claim 10 or vector of claim 11 or host cell of claim 12, for use in a method for treatment or prevention of a cancer, when administered simultaneously or sequentially with an additional cancer therapy, such as a cytokine therapy, a T-cell therapy, an NK therapy, an immune system checkpoint inhibitor, chemotherapy, radiotherapy, immunostimulating substances, gene therapy, antibodies and dendritic cells.

23. The peptide fragment, nucleic acid, vector or host cell of claim 22 wherein the checkpoint blocking antibodies are selected from Actimide, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Etoposide, Fludarabine, Fluorouracil, Gemcitabine, Hydroxyurea, Idarubicin, Irinotecan, Lenalidomide, Leucovorin, Mechlorethamine, Melphalan, Mercaptopurine, Methotrexate, Mitoxantrone, Nivolumab, Oxaliplatin, Paclitaxel, Pembrolizumab, Pemetrexed, Revlimid, Temozolomide, Teniposide, Thioguanine, Valrubicin, Vinblastine, Vincristine, Vindesine and Vinorelbine.
